(19) **Europäisches Patentamt**
**European Patent Office**
**Office européen des brevets**

(11) **EP 3 248 607 B1**

(12) **EUROPEAN PATENT SPECIFICATION**

(45) Date of publication and mention
of the grant of the patent:
**19.11.2025 Bulletin 2025/47**

(51) International Patent Classification (IPC):
**A61K 36/48** (2006.01)    **A61K 36/235** (2006.01)
**A61K 36/185** (2006.01)

(21) Application number: **17172422.2**

(22) Date of filing: **23.05.2017**

(52) Cooperative Patent Classification (CPC):
(C-Sets available)
**A61K 36/53; A61K 36/185; A61K 36/235;
A61K 36/48; A61P 3/10; A61P 9/10; A61P 17/18;
A61P 25/28; A61P 27/12**    (Cont.)

(54) **MAILLARD REACTION PRODUCT-DECOMPOSING AGENT**

MAILLARD-REAKTIONSPRODUKT-ABBAUMITTEL

PRODUIT DE RÉACTION DE MAILLARD - AGENT DE DÉCOMPOSITION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.05.2016 JP 2016102701
22.05.2017 JP 2017100807**

(43) Date of publication of application:
**29.11.2017 Bulletin 2017/48**

(73) Proprietor: **ARKRAY, Inc.**
**Minami-ku
Kyoto-shi,
Kyoto 601-8045 (JP)**

(72) Inventors:
• **Shoshihara, Masako
Kyoto, 602-0008 (JP)**
• **Kawai, Hiroshige
Kyoto, 602-0008 (JP)**

(74) Representative: **Hoffmann Eitle
Patent- und Rechtsanwälte PartmbB
Arabellastraße 30
81925 München (DE)**

(56) References cited:
**EP-A1- 1 683 805    EP-A1- 2 949 362
JP-A- H11 106 336**

• **EDWIN JARALD ET AL: "Diabetes and herbal
medicines", IRANIAN JOURNAL OF
PHARMACOLOGY AND THERAPEUTICS, IRAN
UNIVERSITY OF MEDICAL SCIENCES RAZI
INSTITUTE FOR DRUG RESEARCH, IRAN,
ISLAMIC REPUBLIC OF, vol. 7, no. 1, 1 January
2008 (2008-01-01), pages 97 - 106, XP002656225,
ISSN: 1735-2657**
• **DATABASE BIOSIS [online] BIOSCIENCES
INFORMATION SERVICE, PHILADELPHIA, PA,
US; January 2012 (2012-01-01), RASUL A ET AL:
"Formulation development of a cream containing
fennel extract: in vivo evaluation for anti-aging
effects", XP002774647, Database accession no.
PREV201200211363**
• **PHARMAZIE, vol. 67, no. 1, January 2012
(2012-01-01), pages 54 - 58, ISSN: 0031-7144,
DOI: 10.1691/PH.2012.1083**
• **GURROLA-DIAZ C M ET AL: "Effects of Hibiscus
sabdariffa extract powder and preventive
treatment (diet) on the lipid profiles of patients
with metabolic syndrome (MeSy)",
PHYTOMEDICINE, ELSEVIER, AMSTERDAM, NL,
vol. 17, no. 7, 1 June 2010 (2010-06-01), pages
500 - 505, XP027012957, ISSN: 0944-7113,
[retrieved on 20091203]**

**(Cont. next page)**

(52) Cooperative Patent Classification (CPC): (Cont.)

C-Sets
**A61K 36/185, A61K 2300/00;**
**A61K 36/235, A61K 2300/00;**
**A61K 36/48, A61K 2300/00;**
**A61K 36/53, A61K 2300/00**

**Description**

BACKGROUND OF THE INVENTION

1. Field of the Invention

The present invention relates to a non-therapeutic use of a Maillard reaction product-decomposing agent as defined in the claims.

2. Description of Related Art

It has been known that oxidation reactions and glycation reactions in a living body adversely affect cells and tissues. For example, proteins such as collagen are glycated by a glycation reaction (a Maillard reaction) in a living body and thereby the amino acids contained in the proteins become AGEs (advanced glycation end products) or AGEs form cross-linked structures between proteins. Thus, the accumulation of AGEs in proteins by the Maillard reaction and the formation of cross-linked structures by AGEs cause diseases such as diabetic complications, Alzheimer's disease, cataract, and arteriosclerosis as well as aging and functional decline of tissues such as the skin.
Known drugs for decomposing AGEs include N-phenacylthiazolium bromide (PTB) and N-phenacyl-4,5-dimethylthiazolium bromide (ALT-711) (Sara Vasan et al., Nature, 382, pp275-278 (1996), and Sara Vasan et al., Archives of Biochemistry and Biophysics, 419, 89-96 (2003)). However, they are not used in terms of stability and side effects. In addition, for example, a lotus leaf extract and an Astilbe thunbergii extract have been studied (JP2007-119373A). Further, different extracts including peanut, rosemary, knotweed, leaf lettuce, basil, thyme, spearmint, lemon balm, perilla und rhubarb have been disclosed for the decomposition of AGEs (EP 2 949 362 A1).

SUMMARY OF THE INVENTION

As described above, it has been considered that the accumulation of AGEs associated with the Maillard reaction and the cross-linked structures formed associated therewith may cause various diseases including the aging progress, diabetic complications, etc. Therefore, there are demands for a new substance that can decompose the products formed by the Maillard reaction, such as AGEs and intermediates thereof.
The present invention provides a new non-therapeutic use of a Maillard reaction product-decomposing agent as defined in the claims.
The present invention relates to a non-therapeutic use of a Maillard reaction product-decomposing agent containing an extract of fennel as an active ingredient.

DETAILED DESCRIPTION OF THE INVENTION

The present invention is based on a new finding that water extracts of fennel (scientific name: *Foeniculum vulgare*), lemon balm (scientific name: *Melissa officinalis*), and rosemary (scientific name: *Rosmarinus officinalis*) each have an ability of breaking the cross-linked structures formed by the Maillard reaction, which is higher than that of PTB known as a Maillard reaction cross-link breaker, and particularly, a water extract of fennel, especially a hot water extract thereof, has the highest ability of breaking the cross-linked structures formed by the Maillard reaction.

[Maillard reaction product-decomposing agent]

As an aspect, the present invention relates to a non-therapeutic use of a Maillard reaction product-decomposing agent containing an extract of fennel as an active ingredient (a Maillard reaction product-decomposing agent of the present invention). In one or more embodiments, the non-therapeutic use of a Maillard reaction product-decomposing agent of the present invention can provide an effect of being capable of decomposing a product formed by the Maillard reaction (a Maillard reaction product), preferably an effect of being capable of decomposing a product formed by an amino-carbonyl reaction (also referred to as a Maillard reaction or a glycation reaction) between an amino group of amino acid, peptide, or protein and a sugar-derived carbonyl group in a living body.
In one or more embodiments, the non-therapeutic use of a Maillard reaction product-decomposing agent of the present invention can inhibit the accumulation of AGEs and can break the cross-linked structures of proteins, etc. that are formed by AGEs. Consequently, the diseases, aging of tissues, and functional decline of tissues that are caused by such accumulation and cross-linked structures can be prevented, improved, or treated. In one or more embodiments, the Maillard reaction product-decomposing agent of the present invention can improve metabolism.
Furthermore, the Maillard reaction product-decomposing agent of the present invention can provide an effect of being excellent in safety and productivity since an extract of fennel, which is a plant, is used therein as an active ingredient.

In the present invention, the term "Maillard reaction product" refers to a substance produced by the Maillard reaction. In one or more embodiments, substances produced by the Maillard reaction include an intermediate produced by the Maillard reaction and a final product produced by the Maillard reaction. In one or more embodiments, examples of the Maillard reaction product include polypeptides and proteins containing AGEs, polypeptides and proteins having cross-linked structures formed by AGEs, as well as glycated proteins produced by the Maillard reaction. In one or more embodiments, examples of the intermediate include substances in which the Maillard reaction is in progress and include, as non-limiting examples, glyoxal, methylglyoxal, and 3-deoxyglucosone. In one or more embodiments, examples of AGEs include amino acids and dipeptides glycated by the Maillard reaction, and non-limiting examples thereof include pentosidine, crossline, pyrropyridine, pyrraline, carboxymethyllysine (CML), carboxyethyllysine, carboxymethylarginine (CMA), argpyrimidine, an imidazolone compound, glyoxal-lysine dimer (GOLD), and methyl glyoxal-lysine dimer (MOLD).

In one or more embodiments, the Maillard reaction product-decomposing agent of the present invention provides an effect of allowing a Maillard reaction product to be decomposed. In the present invention, "decomposing a Maillard reaction product" includes decomposing intermediates formed associated with the Maillard reaction or breaking cross-linked structures formed between polypeptides and/or proteins by AGEs. In one or more embodiments, examples of "decomposing a Maillard reaction product" may further include decomposing polypeptides and proteins that contain AGEs, polypeptides and proteins having cross-linked structures formed by AGEs, or glycated proteins produced by the Maillard reaction. Furthermore, in one or more embodiments that are not particularly limited, examples of "decomposing a Maillard reaction product" may include breaking (cleaving) the C-C bonds in diketone structures contained in polypeptides and proteins that contain AGEs, polypeptides and proteins having cross-linked structures formed by AGEs, or glycated proteins produced by the Maillard reaction.

Furthermore, in one or more embodiments, the Maillard reaction product-decomposing agent of the present invention can decompose collagen cross-link (detrimental cross-link or aging cross-link) formed in a collagen molecule by, for example, glycation or oxidation. Thus, in one or more embodiments, the Maillard reaction product-decomposing agent of the present invention can inhibit the accumulation of AGEs in collagen.

In one or more embodiments, the Maillard reaction product-decomposing agent of the present invention may further contain, as an active ingredient, an extract of at least one of lemon balm and rosemary in terms of further improving the ability of breaking the cross-linked structure formed by the Maillard reaction. Moreover, preferably the Maillard reaction product-decomposing agent of the present invention contains at least one of lemon balm and fenugreek in terms of promoting the breakage of the collagen cross-link.

In one or more embodiments, the Maillard reaction product-decomposing agent of the present invention may further contain, as an active ingredient, an extract of at least one of fenugreek (scientific name: *Trigonella foenum-graecum*) and hibiscus (scientific name: *Hibiscus sabdariffa*).

When the Maillard reaction product-decomposing agent of the present invention contains an extract of another plant in addition to fennel, they may be blended at the same ratio (1 part by weight with respect to 1 part by weight of the extract of fennel) or they may be blended at different ratios.

In one or more embodiments, the Maillard reaction product-decomposing agent of the present invention contains an extract of fennel and an extract of lemon balm. In the present aspect, the blending ratio (the dry weight ratio) of the extract of fennel and the extract of lemon balm is, per 1 part by weight of the extract of fennel, 0.1 to 10 parts by weight of the extract of lemon balm, and 0.2 to 5 parts by weight of the extract of lemon balm in terms of further improving the ability of breaking the cross-linked structure formed by the Maillard reaction and promoting the breakage of the collagen cross-link.

In one or more embodiments, the Maillard reaction product-decomposing agent of the present invention contains an extract of fennel and an extract of rosemary. In the present aspect, the blending ratio (the dry weight ratio) of the extract of fennel and the extract of rosemary is, per 1 part by weight of the extract of fennel, 0.1 to 10 parts by weight of the extract of rosemary, and 0.2 to 5 parts by weight of the extract of rosemary in terms of further improving the ability of breaking the cross-linked structure formed by the Maillard reaction.

In one or more embodiments, the Maillard reaction product-decomposing agent of the present invention contains an extract of fennel and an extract of fenugreek. In the present aspect, the blending ratio (the dry weight ratio) of the extract of fennel and the extract of fenugreek is, per 1 part by weight of the extract of fennel, 0.1 to 10 parts by weight of the extract of fenugreek, and 0.2 to 5 parts by weight of the extract of fenugreek in terms of promoting the breakage of the collagen cross-link.

In one or more embodiments, the Maillard reaction product-decomposing agent of the present invention contains an extract of fennel, an extract of lemon balm, an extract of rosemary, and an extract of fenugreek. In the present aspect, the blending ratio (the dry weight ratio) of the extract of fennel, the extract of lemon balm, the extract of rosemary, and the extract of fenugreek is, per 1 part by weight of the extract of fennel, 0.05 to 20 parts by weight of the extract of lemon balm, 0.05 to 20 parts by weight of the extract of rosemary and 0.05 to 20 parts by weight of the extract of fenugreek, and 0.1 to 10 parts by weight of the extract of lemon balm, 0.1 to 10 parts by weight of the extract of rosemary and 0.1 to 10 parts by weight of the extract of fenugreek in terms of further improving the ability of breaking the cross-linked structure formed by the Maillard reaction and promoting the breakage of the collagen cross-link.

The extracts may be extracted from any parts of plants. In one or more embodiments, examples of the part to be used include whole plants, flowers, leaves, seeds, fruits, branches, stems, roots, calyxes, and bracts. The part to be used may be of one type, or two or more types of parts may be combined for use. In one or more embodiments, fennel is preferably extracted from seeds. In one or more embodiments, rosemary is preferably extracted from leaves. In one or more embodiments, lemon balm is preferably extracted from leaves. In one or more embodiments, fenugreek is preferably extracted from seeds. In one or more embodiments, hibiscus is preferably extracted from calyxes and bracts.

The extraction method is not particularly limited, but in one or more embodiments, examples thereof include solvent extraction. In one or more embodiments, examples of the extractant (solvent) include water, alcohols such as methanol and ethanol, and ether. In one or more embodiments, examples of the extract include a water extract and an organic solvent extract. In one or more embodiments, examples of the water extract include a hot water extract. In one embodiment, the solvent extracting is performed at a temperature of 60-100°C, preferably 70-90°C such as 80°C, for a time period of 30 minutes to 5 hours, preferably 2-6 hours or 3-5 hours such as 1 hour. In a preferred embodiment, hot water extraction is carried out at a temperature of 60-100°C, preferably 70-90°C such as 80°C, for a time period of 30 minutes to 5 hours, preferably 2-6 hours or 3-5 hours such as 1 hour. In one or more embodiments, examples of the form of the extract include an aqueous solution, a concentrated liquid, and a dried product. Drying is carried out by heat-treating the extract obtained by the solvent-extracting at a temperature of 70-150°C such as 110°C for 1-10 hours such as 4 hours. A suitable method for producing a Maillard reaction product-decomposing agent according to the present invention is as follows: A defined amount of dry powder of fennel (such as 2 g) is extracted with a suitable amount of distilled water (such as 40 mL) at a temperature of 80°C for one hour, followed by cooling to normal temperature. Thereafter, the obtained slurry is filtrated and the filtrate thus obtained is used as a plant extract. If a dried plant extract shall be used, the obtained plant extract is put into an aluminum tray and dried for e.g. four hours in an incubator set at e.g. 110°C. The form of the Maillard reaction product-decomposing agent of the present disclosure is not particularly limited, but in one or more embodiments, examples thereof include solids, granules, powders, pastes, and liquids.

In one or more embodiments, the Maillard reaction product-decomposing agent of the present invention may contain other extracts and other components as long as they do not inhibit the AGEs decomposing functions of the active ingredients.

In one or more embodiments, the Maillard reaction product-decomposing agent of the present invention can be used as a raw material for a beverage composition, a food composition, a functional food, a cosmetic, or a supplement. The form of the raw material is not particularly limited, but in one or more embodiments, examples thereof include solids, granules, powders, pastes, and liquids.

As one or more embodiments, the present invention relates to the non-therapeutic use of the Maillard reaction product-decomposing agent of the present invention, wherein the fennel extract is comprised as an active agent in a beverage composition, a food composition, a functional food, a cosmetic, or a supplement.

The intake per day of the Maillard reaction product-decomposing agent of the present invention is not particularly limited, but in one or more embodiments, it is at least 5 mg or not more than 2000 mg.

[Method of producing Maillard reaction product-decomposing agent]

In another aspect, the present disclosure relates to a method of producing a Maillard reaction product-decomposing agent for decomposing a Maillard reaction product, the method including extracting fennel with a solvent. According to the production method of the present disclosure, the Maillard reaction product-decomposing agent of the present disclosure can be produced.

In one or more embodiments, the method of producing a Maillard reaction product-decomposing agent of the present disclosure may further include extracting at least one plant selected from the group consisting of lemon balm, rosemary, fenugreek, and hibiscus with a solvent.

In one or more embodiments, the production method of the present disclosure may include solvent-extracting a dried plant.

In one or more embodiments, the production method of the present disclosure may include filtrating or centrifuging an extract obtained by solvent-extracting.

In one or more embodiments, the production method of the present disclosure may include drying a resultant extract.

In one or more embodiments, the production method of the present disclosure may include mixing an extract of fennel and an extract of a plant other than fennel to obtain a mixture of the extracts. In one or more embodiments, the mixing ratios thereof may be the same (1 part by weight with respect to 1 part by weight of the extract of fennel) or may be different from each other.

In one or more embodiments, the production method of the present disclosure may include mixing an extract of fennel and an extract of lemon balm so that the extract of lemon balm is 0.1 to 10 parts by weight per 1 part by weight of the extract of fennel, and in terms of producing an Maillard reaction product-decomposing agent that can further improve the ability of breaking the cross-linked structure formed by the Maillard reaction and/or can promote the breakage of the collagen cross-link, the production method of the present disclosure may include mixing these extracts so that the extract of lemon balm is 0.2 to 5 parts by weight per 1 part by weight of the extract of fennel.

In one or more embodiments, the production method of the present disclosure may include mixing an extract of fennel and an extract of rosemary so that the extract of rosemary is 0.1 to 10 parts by weight per 1 part by weight of the extract of fennel, and in terms of producing an Maillard reaction product-decomposing agent that can further improve the ability of breaking the cross-linked structure formed by the Maillard reaction, the production method of the present disclosure may include mixing these extracts so that the extract of rosemary is 0.2 to 5 parts by weight per 1 part by weight of the extract of fennel.

In one or more embodiments, the production method of the present disclosure may include mixing an extract of fennel and an extract of fenugreek so that the extract of fenugreek is 0.1 to 10 parts by weight per 1 part by weight of the extract of fennel, and in terms of producing an Maillard reaction product-decomposing agent that can promote the breakage of the collagen cross-link, the production method of the present disclosure may include mixing these extracts so that the extract of fenugreek is 0.2 to 5 parts by weight per 1 part by weight of the extract of fennel.

In one or more embodiments, the production method of the present disclosure may include mixing an extract of fennel, an extract of lemon balm, and an extract of rosemary so that, per 1 part by weight of the extract of fennel, the extract of lemon balm is 0.05 to 20 parts by weight, the extract of rosemary is 0.05 to 20 parts by weight and the extract of fenugreek is 0.05 to 20 parts by weight, and in terms of producing an Maillard reaction product-decomposing agent that can further improve the ability of breaking the cross-linked structure formed by the Maillard reaction and/or can promote the breakage of the collagen cross-link, the production method of the present disclosure may include mixing these extracts so that, per 1 part by weight of the extract of fennel, the extract of lemon balm is 0.1 to 10 parts by weight, the extract of rosemary is 0.1 to 10 parts by weight and the extract of fenugreek is 0.1 to 10 parts by weight.

In one or more embodiments, the production method of the present disclosure may include mixing fennel and a plant other than fennel and solvent-extracting the resultant mixture to obtain a mixed extract. In one or more embodiments, the mixing ratios thereof may be the same (1 part by weight with respect to 1 part by weight of the fennel) or may be different from each other. Furthermore, they may be mixed together so that the ratios of the extracts of the respective plants in the resultant mixed extract are the same (1 part by weight with respect to 1 part by weight of the extract of fennel) or they may be mixed together so that the ratios thereof are different from each other.

[Method of producing beverage composition, food composition, functional food, cosmetic, or supplement]

In another aspect, the present disclosure relates to a method of producing a beverage composition, a food composition, a functional food, a cosmetic, or a supplement (a method of producing a beverage composition, etc. of the present disclosure). The method of producing a beverage composition, etc. of the present disclosure includes adding an extract of fennel to a beverage composition, a food composition, a functional food, a cosmetic, or a supplement to provide it with a Maillard reaction product-decomposing effect.

In one or more embodiments, the method of producing a beverage composition, etc. of the present disclosure may further include adding an extract of at least one plant selected from the group consisting of lemon balm, rosemary, fenugreek, and hibiscus to a beverage composition, a food composition, a functional food, a cosmetic, or a supplement to provide it with a Maillard reaction product-decomposing effect.

In one or more embodiments, examples of the extract include a water extract and an organic solvent extract of each plant mentioned above. In one or more embodiments, examples of the water extract include a hot water extract. In one or more embodiments, examples of the form of the extract include an aqueous solution, a concentrated liquid, and a dried product.

[Extract composition]

In another aspect, the present disclosure relates to a fennel extract composition for enhancing the Maillard reaction product-decomposing function. In the present disclosure, the term "extract composition" refers to a liquid or a solid that is obtained by an extraction treatment of a plant. In one or more embodiments, examples of the extract composition include a supernatant and a filtrate that are obtained by solvent-extracting a plant. In one or more embodiments, examples of the extractant include water, alcohols such as methanol and ethanol, and ether. In one or more embodiments, examples of the form of the extract composition include an aqueous solution, a concentrated liquid, and a dried product. In one or more embodiments, the extract composition of the present disclosure can be used as a food raw material. In the present disclosure, the term "food raw material" refers to an ingredient or a raw material that is used for producing, for example, foods such as processed foods and health foods as well as supplements. In one or more embodiments, examples of the form of the food raw material include an aqueous solution, a concentrated liquid, and a dried product.

In one or more embodiments, the extract composition of the present disclosure may contain an extract of at least one plant selected from the group consisting of lemon balm, rosemary, fenugreek, and hibiscus.

[Method of decomposing Maillard reaction product]

In an aspect, the present disclosure relates to a method of decomposing a Maillard reaction product, the method including administering an extract of fennel to a living body. In one or more embodiments, the method of decomposing a Maillard reaction product of the present disclosure may further include administering, to a living body, an extract of at least one plant selected from the group consisting of lemon balm, rosemary, fenugreek, and hibiscus. In an aspect, the present disclosure relates to a method of decomposing a Maillard reaction product, the method including allowing a human or a non-human organism to take in an extract of fennel. In one or more embodiments, the method of decomposing a Maillard reaction product of the present disclosure may use, instead of the above-mentioned extract, a Maillard reaction product-decomposing agent, a beverage composition, a food composition, a functional food, a cosmetic, or a supplement of the present disclosure.

Hereinafter, the present disclosure is further described using examples.

Examples

[Preparation of plant extracts]

After 2 g of dry powder of each plant indicated in Table 1 below was extracted with 40 ml of distilled water at 80°C for one hour, it was cooled to a normal temperature. Thereafter, it was filtrated and the filtrate thus obtained was used as a plant extract.

After 5 mL of resultant filtrate was put into an aluminum tray and then was dried for four hours in an incubator set at 110°C, the remaining amount thereof was measured to calculate the solid content concentration of each sample. The results are indicated in Table 1 below.

[AGEs cross-link breaking effect confirmatory test 1]

The test was carried out according to the description in Sara Vasan et al. (Nature, 382, pp275-278 (1996)).

As a substrate, 1-phenyl-1,2-propanedione (PPD), which is a reaction substrate for an AGEs cross-linking model having an $\alpha$-diketone structure, was used while N-phenacylthiazolium bromide (PTB) was used as a positive control. A plant extract, 10 mmol/L of PPD, and 0.2 mol/L of phosphate buffer solution (pH 7.4) were mixed together at a ratio of 5:1:4 and then were reacted at 37°C for eight hours (n=3). After completion of the reaction, hydrochloric acid was added thereto to stop the reaction.

The reaction solution was centrifuged at 20 °C and 3,000×g for ten minutes and then the amount of benzoic acid contained in the supernatant was analyzed by reverse-phase HPLC. The amount of benzoic acid contained in the reaction solution was determined by subtracting the amount of benzoic acid contained in the sample, which was measured separately. Since 1 mol of PPD produces 1 mol of benzoic acid, the cross-link breaking rate was calculated by the following formula. The results are indicated in Table 1 below.

$$\text{Cross-link breaking rate (\%)} = \{(\text{A-B})/\text{C}\} \times 100$$

A: The amount of benzoic acid contained in the reaction solution
B: The amount of benzoic acid contained in the sample
C: The amount of PPD provided for the reaction (the amount of the substrate)

As a control, a test was carried out using 10 mmol/L of PTB instead of the plant extract.

Table 1

| Common Name (Part to be Used) | Solid Content Concentration (mg/mL) | Cross-Link Breaking Rate (%) (Mean Value ± Standard Deviation) |
|---|---|---|
| Fennel (Seeds) | 1.15 | 39.00 ± 4.30 |
| Lemon Balm (Leaves) | 1.32 | 29.60 ± 2.14 |
| Rosemary (Leaves, Stems) | 1.11 | 26.58 ± 0.25 |

(continued)

| Common Name (Part to be Used) | Solid Content Concentration (mg/mL) | Cross-Link Breaking Rate (%) (Mean Value ± Standard Deviation) |
|---|---|---|
| PTB | - | 20.47 ± 0.29 |

As indicated in Table 1, fennel, lemon balm, and rosemary each exhibited a higher cross-link breaking rate than that of PTB that has been reported as a compound that breaks cross-linked structures associated with AGEs. Particularly, fennel exhibited a cross-link breaking rate nearly twice as high as that of PTB.

[AGEs cross-link breaking effect confirmatory test 2]

The test was carried out according to the description in Sara Vasan et al. (Nature, 382, pp275-278 (1996)).

300 μL/well of SuperBlock T20 (PBS) Blocking Buffer was added to a commercially available 96-well microplate precoated with collagen, which then was reacted at 37°C for one hour. After the reaction, it was washed with 0.05% polyethylene (20) sorbitan monolaurate (Tween 20)/PBS(-), and then AGEs modified bovine serum albumin (AGE-BSA) was added to each well of the microplate, which then was subjected to a crosslinking reaction at 37°C for four hours. After the reaction, it was washed with Tween 20/PBS(-). Thereafter, a plant extract was added to each well and thereby it was subjected to a cross-link breaking (cleaving) reaction at 37°C for 18 hours. After that, it was washed with Tween 20/PBS(-) and a primary antibody (anti-albumin bovine serum rabbit-polyclonal) was added thereto, which then was reacted at room temperature for 30 minutes. Further, after it was washed with Tween 20/PBS(-), a secondary antibody (goat anti-rabbit IgG horseradish peroxidase (HRP) conjugate) was added thereto, which then was reacted at room temperature for 30 minutes. Thereafter, it was washed with Tween 20/PBS(-), and TMB One Component HRP Microwell Substrate was added thereto. Furthermore, a PEG6000 solution was added thereto, which then was reacted for 20 minutes. As a reaction stop solution, 1N HCl was added thereto, and the absorbance at 450 nm (dominant wavelength) / 630 nm (complementary wavelength) was measured with a microplate reader. The amount of AGE-BSA that remained was determined from a calibration curve in which the amount of AGE-BSA was varied. Furthermore, the AGE-BSA decomposition rate (Collagen cross-link decomposition rate) was determined using the following formula. The results are indicated in Table 2 below.

AGE-BSA decomposition rate = {(Amount of AGE-BSA added - Amount of remaining AGE-BSA) / Amount of AGE-BSA added} × 100

As a positive control, the same test was carried out using, instead of the plant extract, punicalagin that has been known to have a Maillard reaction activity-inhibiting effect.

Table 2

| Common Name (Part to be Used) | Amount of Remaining AGE-BSA (ng) | Collagen Cross-Link Decomposition Rate (%) |
|---|---|---|
| Fennel (Seeds) | 812.4 | 18.8 |
| Lemon Balm (Leaves) | 373 | 62.7 |
| Fenugreek (Seeds) | 818.8 | 18.1 |
| Punicalagin | 343.3 | 65.7 |

As shown in Table 2, it was confirmed that fennel, lemon balm, and fenugreek each have a collagen cross-link breaking activity. It was confirmed that in particular, lemon balm has a collagen cross-link breaking activity equivalent to that of punicalagin.

**Claims**

1. Non-therapeutic use of a Maillard reaction product-decomposing agent, comprising an extract of fennel as an active ingredient, for decomposition of a Maillard reaction product, which is decomposing intermediates formed associated with the Maillard reaction or breaking cross-linked structures formed between polypeptides and/or proteins by advanced glycation end products, wherein the extract is a water extract.

2. The non-therapeutic use according to claim 1, wherein the Maillard reaction product-decomposing agent further comprises an extract of at least one plant selected from the group consisting of lemon balm, rosemary, fenugreek and hibiscus.

3. The non-therapeutic use according to claim 1 or 2, wherein the extract is a hot water extract, preferably the extract obtainable by the method defined in claim 9 or 11.

4. The non-therapeutic use according to any one of claims 1 to 3, wherein the Maillard reaction product-decomposing agent comprises, as an active ingredient, a dried product of the extract.

5. The non-therapeutic use according to claim 4, wherein the Maillard reaction product-decomposing agent comprises an extract of fennel and an extract of lemon balm in a dry weight ratio of 0.1 to 10 parts by weight of the extract of lemon balm per 1 part by weight of the extract of fennel.

6. The non-therapeutic use according to claim 4, wherein the Maillard reaction product-decomposing agent comprises an extract of fennel and an extract of rosemary in a dry weight ratio of 0.1 to 10 parts by weight of the extract of rosemary per 1 part by weight of the extract of fennel.

7. The non-therapeutic use according to claim 4, wherein the Maillard reaction product-decomposing agent comprises an extract of fennel and an extract of fenugreek in a dry weight ratio of 0.1 to 10 parts by weight of the extract of fenugreek per 1 part by weight of the extract of fennel.

8. The non-therapeutic use according to claim 4, wherein the Maillard reaction product-decomposing agent comprises an extract of fennel, an extract of lemon balm, an extract of rosemary, and an extract of fenugreek in a dry weight ratio 0.05 to 20 parts by weight of the extract of lemon balm, 0.05 to 20 parts by weight of the extract of rosemary and 0.05 to 20 parts by weight of the extract of fenugreek per 1 part by weight of the extract of fennel.

9. The non-therapeutic use according to any one of claims 1 to 8, wherein the Maillard reaction product-decomposing agent is obtainable by a method comprising solvent-extracting fennel, wherein the solvent extracting is preferably performed at 60-100°C such as 80°C for 30 minutes to 5 hours such as 1 hour.

10. The non-therapeutic use according to claim 9, wherein the method further comprises solvent-extracting at least one plant selected from the group consisting of lemon balm, rosemary, fenugreek and hibiscus.

11. The non-therapeutic use according to any one of claims 9 or 10, wherein the method further comprises drying the extract obtained by the solvent-extracting at a temperature of 70-150°C such as 110°C for 1-10 hours such as 4 hours.

12. The non-therapeutic use according to any one of claims 1 to 8, wherein the fennel extract is comprised as an active agent in a beverage composition, a food composition, a functional food, a cosmetic or a supplement, .

13. The non-therapeutic use according to claim 12, wherein the beverage composition, the food composition, the functional food, the cosmetic or the supplement is produced by a method comprising adding an extract of fennel to a beverage composition, a food composition, a functional food, a cosmetic or a supplement in order to provide it with a Maillard reaction product-decomposing effect.

14. The non-therapeutic use according to claim 13, wherein the method further comprises adding an extract of at least one of lemon balm and rosemary to the beverage composition, the food composition, the functional food, the cosmetic or the supplement.

**Patentansprüche**

1. Nicht-therapeutische Verwendung eines Maillard-Reaktionsprodukt-Zersetzungsmittels, umfassend einen Extrakt aus Fenchel als Wirkstoff, zur Zersetzung eines Maillard-Reaktionsprodukts, das mit der Maillard-Reaktion verbundene Zwischenprodukte zersetzt oder vernetzte Strukturen aufbricht, die zwischen Polypeptiden und/oder Proteinen durch fortgeschrittene Glykationsendprodukte gebildet werden, wobei der Extrakt ein Wasserextrakt ist.

2. Nicht-therapeutische Verwendung gemäß Anspruch 1, wobei das Maillard-Reaktionsprodukt-Zersetzungsmittel ferner umfasst einen Extrakt aus mindestens einer Pflanze, ausgewählt aus der Gruppe, bestehend aus Zitronenmelisse, Rosmarin, Bockshornklee und Hibiskus.

3. Nicht-therapeutische Verwendung gemäß Anspruch 1 oder 2, wobei der Extrakt ein Heißwasserextrakt ist, vorzugsweise der Extrakt, der durch das in Anspruch 9 oder 11 definierte Verfahren erhältlich ist.

4. Nicht-therapeutische Verwendung gemäß mindestens einem der Ansprüche 1 bis 3, wobei das Maillard-Reaktionsprodukt-Zersetzungsmittel umfasst als Wirkstoff ein getrocknetes Produkt des Extrakts.

5. Nicht-therapeutische Verwendung gemäß Anspruch 4, wobei das Maillard-Reaktionsprodukt-Zersetzungsmittel umfasst einen Extrakt aus Fenchel und einen Extrakt aus Zitronenmelisse in einem Trockengewichtsverhältnis von 0,1 bis 10 Gewichtsteilen des Extrakts aus Zitronenmelisse pro 1 Gewichtsteil des Extrakts aus Fenchel.

6. Nicht-therapeutische Verwendung gemäß Anspruch 4, wobei das Maillard-Reaktionsprodukt-Zersetzungsmittel umfasst einen Extrakt aus Fenchel und einen Extrakt aus Rosmarin in einem Trockengewichtsverhältnis von 0,1 bis 10 Gewichtsteilen des Extrakts aus Rosmarin pro 1 Gewichtsteil des Extrakts aus Fenchel.

7. Nicht-therapeutische Verwendung gemäß Anspruch 4, wobei das Maillard-Reaktionsprodukt-Zersetzungsmittel umfasst einen Extrakt aus Fenchel und einen Extrakt aus Bockshornklee in einem Trockengewichtsverhältnis von 0,1 bis 10 Gewichtsteilen des Extrakts aus Bockshornklee pro 1 Gewichtsteil des Extrakts aus Fenchel.

8. Nicht-therapeutische Verwendung gemäß Anspruch 4, wobei das Maillard-Reaktionsprodukt-Zersetzungsmittel umfasst einen Extrakt aus Fenchel, einen Extrakt aus Zitronenmelisse, einen Extrakt aus Rosmarin und einen Extrakt aus Bockshornklee in einem Trockengewichtsverhältnis von 0,05 bis 20 Gewichtsteilen des Extrakts aus Zitronenmelisse, 0,05 bis 20 Gewichtsteilen des Extrakts aus Rosmarin und 0,05 bis 20 Gewichtsteilen des Extrakts aus Bockshornklee pro 1 Gewichtsteil des Extrakts aus Fenchel.

9. Nicht-therapeutische Verwendung gemäß mindestens einem der Ansprüche 1 bis 8, wobei das Maillard-Reaktionsprodukt-Zersetzungsmittel durch ein Verfahren erhältlich ist, umfassend Lösungsmittelextraktion von Fenchel, wobei die Lösungsmittelextraktion vorzugsweise bei 60 bis 100 °C, beispielsweise 80 °C, für 30 Minuten bis 5 Stunden, beispielsweise 1 Stunde, durchgeführt wird.

10. Nicht-therapeutische Verwendung gemäß Anspruch 9, wobei das Verfahren ferner umfasst Lösungsmittelextraktion mindestens einer Pflanze, ausgewählt aus der Gruppe, bestehend aus Zitronenmelisse, Rosmarin, Bockshornklee und Hibiskus.

11. Nicht-therapeutische Verwendung gemäß mindestens einem der Ansprüche 9 oder 10, wobei das Verfahren ferner umfasst das Trocknen des Extrakts erhalten durch die Lösungsmittelextraktion bei einer Temperatur von 70 bis 150 °C, beispielsweise 110 °C, für 1 bis 10 Stunden, beispielsweise 4 Stunden.

12. Nicht-therapeutische Verwendung gemäß mindestens einem der Ansprüche 1 bis 8, wobei der Fenchelextrakt als Wirkstoff in einer Getränkezusammensetzung, einer Lebensmittelzusammensetzung, einem funktionellen Lebensmittel, einem Kosmetikum oder einem Nahrungsergänzungsmittel enthalten ist.

13. Nicht-therapeutische Verwendung gemäß Anspruch 12, wobei die Getränkezusammensetzung, die Lebensmittelzusammensetzung, das funktionelle Lebensmittel, das Kosmetikum oder das Nahrungsergänzungsmittel durch ein Verfahren hergestellt wird, umfassend das Hinzufügen eines Fenchelextrakts zu einer Getränkezusammensetzung, einer Lebensmittelzusammensetzung, einem funktionellen Lebensmittel, einem Kosmetikum oder einem Nahrungsergänzungsmittel, um diesem eine Maillard-Reaktionsprodukt-zersetzende Wirkung zu verleihen.

14. Nicht-therapeutische Verwendung gemäß Anspruch 13, wobei das Verfahren ferner umfasst das Hinzufügen eines Extrakts aus mindestens einem von Zitronenmelisse und Rosmarin zu der Getränkezusammensetzung, der Lebensmittelzusammensetzung, dem funktionellen Lebensmittel, dem Kosmetikum oder dem Nahrungsergänzungsmittel.

**Revendications**

1. Utilisation non thérapeutique d'un agent de décomposition de produit issu de la réaction de Maillard, comprenant un extrait de fenouil en tant que principe actif, pour une décomposition d'un produit issu de la réaction de Maillard, qui décompose des intermédiaires formés associés à la réaction de Maillard ou rompt des structures réticulées formées entre des polypeptides et/ou des protéines par des produits finaux de glycation avancée, dans laquelle l'extrait est un extrait d'eau.

2. Utilisation non thérapeutique selon la revendication 1, dans laquelle l'agent de décomposition de produit issu de la réaction de Maillard comprend en outre un extrait d'au moins une plante sélectionnée parmi le groupe consistant en la citronnelle, le romarin, le fenugrec et l'hibiscus.

3. Utilisation non thérapeutique selon la revendication 1 ou 2, dans laquelle l'extrait est un extrait d'eau chaude, de préférence l'extrait pouvant être obtenu par le procédé défini dans la revendication 9 ou 11.

4. Utilisation non thérapeutique selon l'une quelconque des revendications 1 à 3, dans laquelle l'agent de décomposition de produit issu de la réaction de Maillard comprend, en tant que principe actif, un produit séché de l'extrait.

5. Utilisation non thérapeutique selon la revendication 4, dans laquelle l'agent de décomposition de produit issu de la réaction de Maillard comprend un extrait de fenouil et un extrait de citronnelle en un rapport pondéral à sec allant de 0,1 à 10 parties en poids de l'extrait de citronnelle pour 1 partie en poids de l'extrait de fenouil.

6. Utilisation non thérapeutique selon la revendication 4, dans laquelle l'agent de décomposition de produit issu de la réaction de Maillard comprend un extrait de fenouil et un extrait de romarin en un rapport pondéral à sec allant de 0,1 à 10 parties en poids de l'extrait de romarin pour 1 partie en poids de l'extrait de fenouil.

7. Utilisation non thérapeutique selon la revendication 4, dans laquelle l'agent de décomposition de produit issu de la réaction de Maillard comprend un extrait de fenouil et un extrait de fenugrec en un rapport pondéral à sec allant de 0,1 à 10 parties en poids de l'extrait de fenugrec pour 1 partie en poids de l'extrait de fenouil.

8. Utilisation non thérapeutique selon la revendication 4, dans laquelle l'agent de décomposition de produit issu de la réaction de Maillard comprend un extrait de fenouil, un extrait de citronnelle, un extrait de romarin, et un extrait de fenugrec en un rapport pondéral à sec allant de 0,05 à 20 parties en poids de l'extrait de citronnelle, 0,05 à 20 parties en poids de l'extrait de romarin et 0,05 à 20 parties en poids de l'extrait de fenugrec pour 1 partie en poids de l'extrait de fenouil.

9. Utilisation non thérapeutique selon l'une quelconque des revendications 1 à 8, dans laquelle l'agent de décomposition de produit issu de la réaction de Maillard peut être obtenu par un procédé comprenant une extraction de fenouil par solvant, dans laquelle l'extraction par solvant est effectuée de préférence à 60-100°C, par exemple à 80°C, pendant 30 minutes à 5 heures, par exemple 1 heure.

10. Utilisation non thérapeutique selon la revendication 9, dans laquelle le procédé comprend en outre une extraction par solvant d'au moins une plante sélectionnée parmi le groupe consistant en la citronnelle, le romarin, le fenugrec et l'hibiscus.

11. Utilisation non thérapeutique selon l'une quelconque des revendications 9 ou 10, dans laquelle le procédé comprend en outre un séchage de l'extrait obtenu par l'extraction par solvant à une température de 70-150°C, par exemple à 110°C, pendant 1-10 heures, par exemple 4 heures.

12. Utilisation non thérapeutique selon l'une quelconque des revendications 1 à 8, dans laquelle l'extrait de fenouil est compris comme agent actif dans une composition de boisson, une composition alimentaire, un aliment fonctionnel, un cosmétique ou un complément.

13. Utilisation non thérapeutique selon la revendication 12, dans laquelle la composition de boisson, la composition alimentaire, l'aliment fonctionnel, le cosmétique ou le complément est produit(e) par un procédé comprenant un ajout d'un extrait de fenouil à une composition de boisson, une composition alimentaire, un aliment fonctionnel, un cosmétique ou un complément afin de lui conférer un effet de décomposition de produit issu de la réaction de Maillard.

14. Utilisation non thérapeutique selon la revendication 13, dans laquelle le procédé comprend en outre un ajout d'un extrait d'au moins une plante parmi la citronnelle et le romarin à la composition de boisson, la composition alimentaire, l'aliment fonctionnel, le cosmétique ou le complément.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007119373 A

- EP 2949362 A1

**Non-patent literature cited in the description**

- **SARA VASAN et al.** *Nature*, 1996, vol. 382, 275-278

- **SARA VASAN et al.** *Archives of Biochemistry and Biophysics*, 2003, vol. 419, 89-96